# EUROPEAN PATENT APPLICATION

(11) **EP 1 637 134 A1**
(43) Date of publication of application: **22.03.2006**
(21) Application number: 04746413.6
(22) Date of filing: 18.06.2004
(51) Int. Cl.: A61K 31/202, A61K 31/232, A61K 8/18, A61P 9/00, A23L 1/30

(54) **COMPOSITION FOR PREVENTION/TREATMENT FOR VARICOSE VEINS**

(30) Priority: 20.06.2003 JP 2003177339
(71) Applicant: MOCHIDA PHARMACEUTICAL CO., LTD., Shinjuku-ku Tokyo 160-8515 (JP)
(72) Inventor: KAKIUCHI, Toshihiko, 8490922 (JP)
(74) Representative: Grünecker, Kinkeldey, Stockmair & Schwanhäusser Anwaltssozietät
(86) International application number: PCT/JP2004/008944
(87) International publication number: WO 2004/112777

(57) **Abstract**

A preventive/therapeutic agent for varicose veins or a composition for the prevention or alleviation of symptoms relevant to varicose veins. The preventive/therapeutic agent for varicose veins or the composition for the prevention or alleviation of symptoms relevant to varicose veins each is **characterized by** containing eicosapentaenoic acid.

## Description

### Technical Field

This invention relates to a composition for preventing or alleviating symptoms associated with varicose veins of lower extremities which contains eicosapentaenoic acid (hereinafter referred to as EPA for abbreviation) as its effective component. This invention also relates to a prophylactic and therapeutic agent for varicose veins of lower extremities.

### Background Art

Varicose veins of lower extremities are the state of abnormal and unattractive dilatation and twisting of subcutaneous veins due to dysfunction of valves in great saphenous vein, small saphenous vein, perforator vein artery, and other subcutaneous veins in the legs, and such valve dysfunction is caused by heredity factors or sustained increase of hydrostatic pressure in the leg veins due to prolonged standing for long hours on the job, pregnancy, manual labor, and the like. Typical treatments are compression therapy (wearing of elastic stockings, elastic bandage, etc.) and surgery (vein stripping, vein sclerotherapy, etc.), and no drug has so far been confirmed to have the effect of preventing and treating the varicose veins of lower extremities. Despite the fact that the varicose veins of lower extremities may in serious cases worsen to the manifestation of skin ulcer, progression of the varicose veins of lower extremities is usually slow, and it is neither painful nor life-threatening, and as a consequence, physicians tend to be less interested in this disease and less accurate in their knowledge, and many patients are currently being left untreated. Even before the stage that could be called a disease, conditions such as "spider veins (veins looking like spider webs) are noticed on the backside of the arm, calf, or knee" and "bulging vein stands out" are manifested, and such conditions are cosmetically unfavorable, especially in the case of a female patient. Accordingly, there is a demand for an effective therapeutic method, and in particular, for both effective drug and composition.

Treatment of the varicose veins of lower extremities has traditionally depended on compression therapy or surgery, and there has been no confirmed effective therapeutic agent. For treating mild varicose veins of lower extremities, a supplement containing aqueous extract of red grape leaf has been disclosed for preventing or alleviating the discomfort associated with mild or moderate chronic leg venous insufficiency (see for example, JP 2001-122791 A). A cream containing pycnogenol which is a component of the bark of the French maritime pine is also on the market, and this cream is said to be effective for the spider veins.

However, these agents all target mild to moderate chronic leg venous insufficiencies such as spider veins which is the stage before being diagnosed as a disease, and there has been no therapeutic agent for varicose veins of lower extremities that can be used for all the range of conditions from mild conditions like spider veins to more advanced conditions such as development of skin ulcer.

### Disclosure of the Invention

Accordingly, an object of the present invention is to provide a drug, and in particular, a drug for oral administration which has prophylactic and therapeutic effects for a wide range of varicose veins of lower extremities exhibiting mild to serious symptoms with reduced side effects; and a composition which prevents or alleviates the symptoms associated with the varicose veins of lower extremities. Another object of the present invention is to provide use of eicosapentaenoic acid in the manufacture of a prophylactic and therapeutic agent for the varicose veins of lower extremities, and a method for preventing and treating the varicose veins of lower extremities using eicosapentaenoic acid.

The inventor of the present invention made an intensive study to find a drug which has prophylactic and therapeutic effects on the varicose veins of lower extremities with reduced side effects, and a composition which prevents or alleviates the symptoms associated with the varicose veins of lower extremities, and found that the prophylactic and therapeutic agent and the composition of the present invention containing EPA as their effective component have such effects.

EPA in the form of ethyl ester has been used as a drug for "alleviating ulcer, pain, and cryesthesia associated with arteriosclerosis obliterans" and "hyperlipidemia". EPA is also known to have pharmacological effects such as reducing of serum lipid, antiplatelet action, improvement of erythrocyte deformability, and suppression of abnormal contraction of smooth muscle. However, there has so far been no report indicating that the EPA has therapeutic effect for the varicose veins of lower extremities.

Varicose veins of lower extremities are caused by valve dysfunctioning of subcutaneous veins such as great saphenous vein, small saphenous vein, and perforator vein in the lower extremities, and the resulting interruption of smooth blood flow and blood reflux in the subcutaneous veins. Therefore, varicose veins of lower extremities cannot be treated, and the symptoms associated with the varicose veins of lower extremities cannot be alleviated by merely improving the blood flow of the subcutaneous veins.

The mechanism for treating the varicose veins of lower extremities and alleviating the symptoms associated with the varicose veins of lower extremities by the administration of the EPA is yet unknown. However, EPA presumably acts in some way or another, and as the outcome, the varicose veins of lower extremities are treated and the symptoms associated with the varicose veins of lower extremities are alleviated.

Accordingly, the present invention based on such findings provides a composition for preventing or alleviating symptoms associated with the varicose veins of lower extremities wherein the composition contains eicosapentaenoic acid.

The composition of the present invention is preferably a food, a dietary supplement, a cosmetic, or a quasi drug.

The present invention also provides a prophylactic and therapeutic agent for the varicose veins of lower extremities which contains eicosapentaenoic acid as its effective component.

The prophylactic and therapeutic agent for the varicose veins of lower extremities according to the present invention preferably contains ethyl ester of the eicosapentaenoic acid as its effective component.

### Best Mode for Carrying Out the Invention

Next, the present invention is described in detail.

First aspect of the present invention relates to a composition for preventing or alleviating symptoms associated with the varicose veins of lower extremities which contains eicosapentaenoic acid (hereinafter occasionally referred to as "the composition of the present invention" for the sake of simplicity). The composition of the present invention is preferably a food, a dietary supplement, a cosmetic, or a quasi drug.

Second aspect of the present invention relates to a prophylactic and therapeutic agent for the varicose veins of lower extremities which contains eicosapentaenoic acid as its effective component (hereinafter occasionally referred to as "the prophylactic and therapeutic agent of the present invention" for simplicity). The prophylactic and therapeutic agent of the present invention preferably contains ethyl ester of the eicosapentaenoic acid as its effective component.

In the present invention, the term "varicose veins of lower extremities" refers to the state of abnormal and unattractive dilatation and twisting of subcutaneous veins due to valve dysfunctioning of great saphenous vein, small saphenous vein, perforator vein, and other subcutaneous veins in the legs, and such valve dysfunctioning is caused by heredity factors or sustained increase of hydrostatic pressure in the leg veins due to prolonged standing for long hours on the job, pregnancy, manual labor, and the like. The term "symptoms associated with the varicose veins of lower extremities" includes painless skin deformation, spider veins, leg edema, tired legs, and the like caused by the varicose veins of lower extremities, but the symptoms are not limited to those indicated above.

The composition or the prophylactic and therapeutic agent of the present invention has therapeutic, alleviating, and prophylactic effects for a wide range of varicose veins of lower extremities of mild to serious conditions. More specifically, the composition or the prophylactic and therapeutic agent of the present invention has therapeutic, alleviating, and prophylactic effects for a wide range of varicose veins of lower extremities and symptoms associated with the varicose veins of lower extremities from relatively mild conditions such as spider veins which is the externally visible dilatation of the subcutaneous veins, leg edema, and tired legs, to more advanced conditions such as swelling of subcutaneous veins into bulged varices, or more advanced conditions wherein ulcer and pigmentation have developed on the skin.

In the present invention, "therapeutic, alleviating, and prophylactic effects for the varicose veins of lower extremities and symptoms associated with the varicose veins of lower extremities" means the effect of preventing development of the varicose veins of lower extremities or symptoms associated with the varicose veins of lower extremities accomplished by taking or administering the composition or the prophylactic and therapeutic agent of the present invention. Heredity factors, occupational factors (prolonged standing), birthing experience, and the like have been confirmed to be the factors that may be relevant to the varicose veins of lower extremities, and therefore, if a person having such factor takes or is administered in advance with the composition or the prophylactic and therapeutic agent of the present invention, such intake or administration is expected to have the effect of preventing the development of the varicose veins of lower extremities and the symptoms associated with the varicose veins of lower extremities.

The EPA used in the composition or the prophylactic and therapeutic agent of the present invention may be a commercially available EPA, or the one produced by purifying fish oil or EPA-producing bacteria or its culture midium by a method known in the art such as continuous distillation, urea adduct method, liquid chromatography, supercritical fluid chromatography, or a combination thereof. If desired, the EPA may be esterified to obtain an alkyl ester such as ethyl ester, a glyceride (for example, triglyceride), or other ester. EPA may also be used as a salt with an inorganic base, for example, as sodium salt or potassium salt, or a salt with an organic base, for example, as benzylamine salt or diethylamine salt, or a salt with a basic amino acid, for example, as arginine salt or lysine salt. Unless otherwise noted, the term "EPA" used in the present invention also includes such salts and esters in addition to the fatty acid in free form. When administered to a human or an animal, the EPA used is preferably the one acceptable in view of pharmaceutics and/or food hygienics.

The composition or the prophylactic and therapeutic agent of the present invention can surely contain a pure EPA product. However, it may also contain a fatty acid other than EPA as its effective component. Exemplary such fatty acids include docosahexaenoic acid (hereinafter abbreviated as DHA), docosapentaenoic acid, docosamonoenoic acid, arachidonic acid, eicosatetraenoic acid, eicosatrienoic acid, eicosamonoenoic acid, octadecatetraenoic acid, α-linolenic acid, linoleic acid, oleic acid, palmitoleic acid, hexadecatetraenoic acid, hexadecatrienoic acid, hexadecadienoic acid, and other unsaturated fatty acids; and behenic acid, arachidic acid, stearic acid, palmitic acid, myristic acid, and other saturated fatty acids.

With regard to the composition of the present invention, it is rather preferable that the composition also contain such fatty acid other than EPA. Since the composition of the present invention is preferably a food, a dietary supplement, a cosmetic, or a quasi drug, the composition may have, and preferably has effects other than the effect of preventing or alleviating the symptoms associated with varicose veins of lower extremities. When the composition of the present invention contains a fatty acid other than the EPA as described above in addition to the EPA, the composition can also benefit from the effects of such fatty acid other than the EPA.

The fatty acid used in the composition of the present invention is preferably a naturally occurring fatty acid. More specifically, sardine oil, squid oil, cod liver oil, menhaden oil, krill oil, herring oil, saury oil, mackerel oil, and the like may be treated by a method known in the art such as deoxidation, decolorization, deodorization, degumming, and dewaxing, optionally followed by solvent fractionation, urea adduct method, molecular distillation, or the like to produce a mixture of EPA and other fatty acids such as DHA concentrated to a certain degree.

With regard to the prophylactic and therapeutic agent, the agent does is preferably substantially free from such fatty acid other than EPA, and in particular, from ω-6 polyunsaturated fatty acid and DHA because it is preferable to effectively develop the prophylactic and therapeutic effect for the varicose veins of lower extremities.

The fatty acid in the embodiment as described above may be the one in free form, or a salt with an inorganic base such as sodium salt, or a salt with an organic base such as benzylamine salt, or its ester such as an alkyl ester, for example, ethyl ester, or glyceride.

The content of the EPA in the total fatty acids in the composition or the prophylactic and therapeutic agent of the present invention is not particularly limited. In the case of the prophylactic and therapeutic agent, however, the content is preferably at least 50% by weight, more preferably at least 70% by weight, still more preferably at least 85% by weight, and most preferably such that the agent is substantially free from the fatty acid component other than the EPA. The effective component is preferably eicosapentaenoic acid ethyl ester (hereinafter abbreviated as EPA-E). In the case of the composition, the content of EPA in the total fatty acids may be 10 to 100% by weight, and preferably 15 to 75% by weight.

In the composition of the present invention, the content of the EPA in relation to the total weight of the composition may be adequately selected depending on the form of the composition. The EPA content, however, is preferably 0.05 to 50% by weight, and more preferably 0.1 to 25% by weight. When the EPA content in relation to the total weight of the composition is within such range, the effect of preventing or treating the symptoms associated with the varicose veins of lower extremities will be achieved, and other properties required for the composition will also be fully retained. For example, when the composition is a food or a dietary supplement, texture, taste, and the like will not be impaired, and when the composition is a cosmetic or a quasi drug, no adverse effect will be exerted on the other properties required for such products.

The composition or the prophylactic and therapeutic agent of the present invention may be administered either as a compound or compounds, namely, as effective ingredients alone, or by preparing an adequate composition or pharmaceutical formulation by combining with an adequate vehicle or a medium commonly used in the art such as an excipient, a binder, a lubricant, a colorant, or a flavor; and in some cases, a sterilized water or a vegetable oil; and further in combination with a non-toxic organic solvent or a non-toxic solubilizer (such as glycerin or propylene glycol), an emulsifier, a suspending agent (for example, Tween 80 and gum arabic solution), an isotonizing agent, a pH adjusting agent, a stabilizer, an analgesic, and the like. The product prepared by such procedure may also be incorporated in a food, a dietary supplement, a cosmetic, or a quasi drug in the course of its production to thereby produce the desired composition.

An unsaturated fatty acid such as EPA suffers from extremely poor oxidation stability, and the oxide formed is likely to cause loss of activity, inferior flavor, and adverse effects on health. Therefore, the composition or the prophylactic and therapeutic agent of the present invention desirably contains an effective amount of an antioxidant or other substance which can suppress oxidation of the EPA and other unsaturated fatty acids, and exemplary such reagents include butylated hydroxytoluene (BHT), butylated hydroxyanisole (BHA), propyl gallate, gallic acid, pharmaceutically acceptable quinone, and α-tocopherol. Use of a lemon, pineapple, or other flavor; spice; cyclodextrin, lecithin, or other masking agent is also preferable. Alternatively, the EPA and other unsaturated fatty acids may be treated so that (i) they are coated with an edible film-forming substance to substantially block their contact with the outer atmosphere (see, for example, JP 59-17949 A); (ii) they are emulsified and dispersed in cyclodextrin-containing solution and then dried to make a product in powder form (see, for example, JP 58-13541 A and JP 60-34156 A); or (iii) they are processed into powder after adding casein, saccharide, or the like (see, for example, JP 60-49097 A). The various methods described in this paragraph may also be used in an adequate combination.

The composition or the prophylactic and therapeutic agent of the present invention may also contain, as an effective component other than the EPA, an aqueous extract of red grape leaf or the flavonoid which is the effective component of the extract, an extract of the bark of the French maritime pine or pycnogenol which is the effective component of the extract, horse chestnut extract, or hazel extract. It may also contain a component such as lecithin that promotes absorption of the EPA and other unsaturated fatty acids.

The composition or the prophylactic and therapeutic agent of the present invention are administered in the forms or in the dosage forms of tablet, capsule, microcapsule, granule, fine granule, powder, liquid preparation for oral administration, suppository, syrup, inharant, eye drop, ointment, injection (emulsifying, suspending, or non-aqueous), and solid injection which is emulsified or suspended immediately before its use. The composition or the agent may be administered to the patient, for example, orally, intravenously, intraarterially, by inhalation, by instillation from eye, intrarectally, intravaginally, or externally. The preferred form is capsule such as soft capsule or microcapsule which is orally administered. Also preferred are an injection (emulsifying, suspending, or non-aqueous), and a solid injection which is to be emulsified or suspended immediately before its use for intravenous or intraarterial administration.

The composition of the present invention is preferably a food, a dietary supplement, a cosmetic, or a quasi drug, and accordingly, the composition may be prepared in any desired form appropriate for its application.

When the composition is a food or a dietary supplement, it may be a normal food, or alternatively, a functional food, a specified health food, a nutritional supplementary food, a maternity food, a geriatric food, or the like. Non-limiting examples of the food include bread, Danish pastry, pie, cake, biscuit, cracker, cookie, doughnut, wafer, jam, chocolate, chewing gum, jelly, pudding, candy, lollipop, caramel, and other breads and confectionery; cream, whipped cream, coffee cream, ice cream, butter, cheese, milk beverage, processed milk, yogurt, ham, sausage, and other processed milk and meat products; margarine, shortening, fat spread, mayonnaise, dressing, cooking oil, salad oil, and other fat and oil food products; miso, soy sauce, sauce, and other seasonings; bean curd, noodle, sprinkles, and other processed foods; beverage powder, soup powder, and other food powders; freshener, sports drink, juice, lactic acid bacteria beverage, alcoholic beverage, tea, soup, soy milk, vitamin and mineral beverage, protein beverage, and other beverages. Non-limiting exemplary forms of the dietary supplement include enteral nutritional supplement, powder, granule, pill, tablet, capsule, troche, liquid medication for internal use, suspension, emulsion, and syrup.

Examples of the cosmetic and the quasi drug include skin lotion, essence, milky lotion, cream, lotion, pack, powder product, body soap, and soap, and the preferred is a cream.

EPADEL and EPADEL S (both being products manufactured by Mochida Pharmaceutical Co., Ltd.) are soft capsules containing high purity EPA-E, and they are highly safe therapeutic agent for arteriosclerosis obliterans and hyperlipidemia commercially available in Japan with few side effects. They can be used in the composition and the prophylactic and therapeutic agent of the present invention.

The composition and prophylactic and therapeutic agent of the present invention may be administered at any amount sufficient for them to exert the desired effect. The amount, however, may be adjusted as appropriate to reflect the dosage form, administration route, number of administration per day, seriousness of the symptom, body weight, age, and the like. For example, in the case of oral administration of a prophylactic and therapeutic agent, the agent is preferably administered at 0.1 to 9 g/day, preferably 0.5 to 6 g/day, and more preferably 1 to 3 g/day in terms of EPA, and the agent may be administered as appropriate in a single dose or in divided doses, and preferably in several doses, and in particular, in about three divided doses. In the case of intravenous or intraarterial administration, the agent is preferably administered at 1 to 200 mg, preferably 5 to 100 mg, and more preferably 10 to 50 mg in terms of EPA, and the agent may be administered as appropriate in a single dose or in divided doses. Also, if necessary, the agent may be administered continuously for several hours to several days by drip infusion, infusion pump, or the like as desired.

### (Examples)

Next, the present invention is described in further detail by referring to Examples, which by no means limit the scope of the present invention.

### Example 1

### [Subject, method, and results]

A 83 year old female diagnosed with hypertension, diabetes, hyperlipidemia, and varicose veins of lower extremities (serious conditions with multiple dilatations, winding varices, and skin pigmentation in both legs) was administered with Dorner (trade name; manufactured by Toray Industries, Inc.; generic name, beraprost sodium; a therapeutic agent for chronic artery occlusion) in expectation of therapeutic effect on varicose veins of lower extremities together with Adalat L (trade name; manufactured by Bayer; generic name, nifedipine; coronary vasodilator), Basen (trade name; manufactured by Takeda Pharmaceutical Company Limited; generic name, voglibose; α-glucosidase inhibitor), Lochol (trade name; manufactured by Ciba Geigy Japan Ltd.; generic name, fluvastatin sodium; HMG-CoA reductase inhibitor), and NU-LOTAN (trade name; manufactured by Banyu Pharmaceutical Co., Ltd.; generic name, losartan potassium; angiotensin II receptor antagonist). No improvement in the varicose veins of lower extremities, however, was observed after the medication. Accordingly, Dorner was replaced with EPADEL S (trade name; manufactured by Mochida Pharmaceutical Co., Ltd.; generic name, ethyl eicosapentate (EPA-E)) at a dose of 600 mg x 3 packets per day. After about 1 month of the new medication, substantial disappearance of the varicose veins in the right leg and substantial improvement of the varicose veins in the left leg were observed. The skin pigmentation also disappeared. No adverse event presumably caused by EPADEL S was observed. No therapeutic method was changed except the change of the drug from Dorner to EPADEL S.

The results as described above confirm that EPA may serve an effective and useful therapeutic agent for the varicose veins of lower extremities.

### [Product Examples]

Next, specific production examples of the composition of the present invention are described.

### (1) Butter cookie

150 g of butter, 250 g of shortening, 80 g of milk, 90 g of sugar and 90 g of egg were thoroughly mixed while being sufficiently stirred with a home food mixer. 330 g of flour, 2.5 g of baking powder, and 5 g of oxidation-resistant EPA were added, and kneading was continued. The resulting dough was allowed to stand for 30 minutes, divided into 50 pieces with a cookie cutter, baked in an oven to produce EPA-containing butter cookies. The content of the EPA in relation to the total weight of the butter cookies was 0.5%.

### (2) Mayonnaise 1

EPA-containing mayonnaise was produced by mixing 8 kg of egg yolk (containing 20% of phospholipid), 70 kg of salad oil, 10 kg of oxidation-resistant EPA, 11 kg of vinegar, 0.8 kg of salt, and 0.2 kg of seasoning in a vacuum mixer. The content of the EPA in relation to the total weight of the mayonnaise was 10%.

### (3) Mayonnaise 2

EPA-containing mayonnaise was produced by a method commonly used in the art from 66 kg of corn oil containing 20% of oxidation-resistant EPA-rich purified fish oil (containing 30% of EPA), 20 kg of egg yolk, and 14 kg of vinegar. The content of the EPA in relation to the total weight of the mayonnaise was 4.0%.

### (4) Ice cream

To 6 kg of oxidation-resistant EPA were added 7.9 kg of skimmilk powder, 20 kg of sugar, 0.2 kg of stearic acid monoglyceride, and 0.2 kg of casein, and water was further added to a total of 100 kg. The mixture was heated to 60°C with stirring. After homogenizing the mixed ingredients in a homogenizer, the mixture was sterilized by heating at 70°C for 30 minutes and immediately cooled to 0°C. The mixture was allowed to stand at the same temperature all day and night, and then cooled to -2°C with vigorous agitation for air incorporation. Finally, the mixture was hardened in a freezer to produce EPA-containing ice cream. The content of the EPA in relation to the total weight of the ice cream was 6%.

### (5) Biscuit

Purified fish oil containing EPA and DHA (EPA content, 20%) was placed in an inner tube of a double tube, and a molten gelatin solution heated to 40 to 50°C was placed between the inner tube and an outer tube of the double tube, and the oil and the gelatin solution were simultaneously introduced into cooled water to produce gelatin-coated oil beads having a diameter of 1 mm.

Next, 100 kg of flour, 20 kg of shortening, 40 kg of sugar, 5 kg of glucose, 3 kg of skimmilk powder, 1 kg of salt, 0.5 kg of sodium bicarbonate, and 20 kg of the gelatin-coated oil beads as described above were kneaded to produce biscuit dough, and this dough was baked at 180°C to produce EPA-containing biscuits. The content of the EPA in relation to the total weight of the biscuits was 2.1%.

### (6) Chewing gum

5 kg of gelatin-coated oil beads having a diameter of 0.5 mm produced by the method similar to the above (5), 22 kg of gum base, 22 kg of calcium carbonate, 15 kg of starch syrup, 60 kg of powdered sugar, and 1 kg of lemon flavor were homogeneously mixed in a blender to produce EPA-containing chewing gum. The content of the EPA in relation to the total weight of the chewing gum was 0.8%.

### (7) Chocolate

To 40 kg of purified fish oil containing EPA and DHA (EPA content, 20%) were added 60 kg of water, 3 kg of casein sodium, and 6 kg of gelatin, and mixture was stirred and homogenized in a homogenizer to produce an oil-in-water type oil emulsion. The resulting oil emulsion was sprayed through an orifice having a diameter of about 0.1 mm into a hot atmosphere at about 120°C to produce fine powder of coated oil. EPA-containing chocolates were then produced by a method commonly used in the art by using 20.5 kg of bitter chocolate, 20 kg of chocolate butter, 17 kg of whole milk powder, 42.5 kg of powdered sugar, 0.45 kg of lecithin, 0.5 kg of vanilla flavor, and 10 kg of the coated oil. The content of the EPA in relation to the total weight of the chocolates was 0.3%.

### (8) Capsule 1

9.7 kg of purified fish oil containing 15% of EPA and 0.5 kg of egg yolk lecithin were placed in an agitation tank, and homogenized until fully uniform liquid mixture of ingredients was formed. In the meanwhile, 60% of gelatin, 30% of glycerin, and 10% of water were mixed and the mixture was formed into a film, which was then injection molded into a capsule container (ellipsoid) with an internal volume of 300 mg. In the capsule container was introduced 300 mg of the liquid ingredient mixture, and the inlet was sealed through heating. Thus, 34,000 capsules of the EPA-containing dietary supplement were produced. The EPA content of each capsule was 14.3%.

### (9) Capsule 2

10 kg of purified fish oil containing 28% of EPA and 0.1 kg of tocopherol were placed in an agitation tank, and agitated until fully uniform liquid mixture of ingredients was formed. In the meanwhile, 2.6 kg of gelatin, 0.9 kg of glycerin, and 1.8 kg of water were mixed and the mixture was formed into a film, which was then injection molded into a capsule container (ellipsoid) with an internal volume of 300 mg. In the capsule container was introduced 300 mg of the liquid ingredient mixture, and the inlet was sealed through heating. Thus, 33,600 capsules of EPA-containing dietary supplement having a total weight of 460 mg were produced. The EPA content of each capsule was 18.1%.

### (10) Milk beverage 1

To 0.5 kg of skimmilk powder dissolved in 96.35 kg of skimmilk was added a separately prepared emulsifier (0.1 kg of monoglyceride and 0.05 kg of sucrose ester), and the mixture was dissolved by warming to 65°C. To this mixture was gradually added 3 kg of corn oil containing 20% of oxidation-resistant EPA-rich purified fish oil (containing 30% of EPA), and the mixture was agitated for emulsification. The mixture was further homogenized to produce an EPA-containing nutritional milk beverage. The content of the EPA in relation to the total weight of the milk beverage was 0.2%.

### (11) Milk beverage 2

To 10 kg of EPA-rich purified fish oil (containing 30% of EPA) was dissolved 100 g of lecithin, and this solution was added dropwise to 10 kg of cyclodextrin-containing solution (21% of cyclodextrin, 46% of other saccharides, and 33% of water) with stirring (at 30 rpm) for emulsification and dispersion. After 7 minutes of the dropwise addition, stirring was continued for another 3 minutes to produce an ivory white paste of the emulsified dispersion.
Next, 3.6 kg of the resulting emulsified dispersion was added to processed milk prepared by blending 120 kg of milk, 55.5 kg of skimmilk and 0.9 kg of skimmilk powder, and the resulting dispersion was homogenized at a pressure of 200 kg/cm², and sterilized at 130°C for 2 seconds to prepare a EPA-containing milk beverage. The content of the EPA in relation to the total weight of the milk beverage was 0.3%.

### (12) Caramel

6 kg of safflower oil containing 20% of oxidation-resistant EPA-rich purified fish oil (containing 30% of EPA) was added to a caramel blend (35 kg of starch syrup, 20 kg of sugar, 35 kg of evaporated milk, 4 kg of flour, and 0.2 kg of salt), and the mixture was boiled down, molded, and cooled to produce EPA-containing caramels. The content of the EPA in relation to the total weight of the caramels was 0.4%.

### (13) Powder 1

To 9 kg of corn oil were blended 1 kg of EPA-rich purified fish oil (containing 30% of EPA) and 0.1 kg of lecithin to produce modified oil. 4 kg of the modified oil was added dropwise to 6 kg of cyclodextrin-containing solution (21% of cyclodextrin, 46% of other saccharides, and 33% of water) with stirring (at 60 rpm) to produce 10kg of emulsified dispersion in 10 minutes.

5 kg of this emulsified dispersion was diluted with 5 kg of water, and the dilution was dried by a spray drier at a rate of 100 ml/min and at a hot air temperature of 130°C, and rapidly cooled to produce powder. This powder could be used as a dietary supplement with no further processing. A favorable nutritional supplementary food was obtained by incorporating 2 to 10% of the powder in soup powder, beverage powder, or ice cream mix powder. The content of the EPA in relation to the total weight of the powder was 4%.

### (14) Powder 2

To 40 g of γ-cyclodextrin were added 40 mL of water and 30 g of high purity EPA-E, and the mixture was kneaded for 15 minutes and dried under reduced pressure for 4 hours at 60°C. The mixture was pulverized to produce about 70 g of a powdery EPA-γ-cyclodextrin inclusion compound. The content of the EPA in relation to the total weight of the powder was 42.9%.

### (15) Carbonated beverage

1.97 kg of commercially available isomerized sugar (isomerization rate, 55%), 15 g of the powdery EPA-γ-cyclodextrin inclusion compound produced in (14), 23 g of citric acid, 0.2 g of vitamin B1-nitrate, and 0.5 g of vitamin B6 were added to 8 L of water, and the mixture was stirred for dissolution. Two volumes of carbon dioxide was incorporated in the mixture with a carbonator by a method well known in the art to thereby produce an EPA-containing carbonated beverage. The content of the EPA in relation to the total weight of the carbonated beverage was 0.06%.

### (16) Tablet

100 g of crystalline maltose in powder form (trade name, Sunmalt; manufactured by Hayashibara Co., Ltd.), 10 g of corn starch, and 10 g of the powdery EPA-γ-CD inclusion compound produced in (14) were uniformly mixed. EPA-containing tablets each having a weight of 680 mg, tablet thickness of 5.25 mm, and hardness of 8 kg ± 1 kg were made by using 20R punches having a diameter of 12 mm. The EPA content per tablet was 3.6%.

### (17) Cream

EPA-containing cream having the formulation of: 5% of beeswax, 5% of cetanol, 20% of squalane, 5% of lipophilic glyceryl monostearate, 2% of polyoxyethylene sorbitan monolaurate, 5% of 1,3-butylene glycol, 2% of EPA, antioxidant (q.s.), antiseptic (q.s.), flavor (q.s.), and purified water (balance) was produced by the method commonly used in the art. The content of the EPA in relation to the total weight of the cream was 2.0%.

### Industrial Applicability

The composition containing eicosapentaenoic acid as its effective component and the prophylactic and therapeutic agent for varicose veins of lower extremities will be an effective composition for preventing or alleviating the symptoms associated with the varicose veins of lower extremities and an effective prophylactic and therapeutic agent for the varicose veins of lower extremities.

## Claims

1. A composition for preventing or alleviating symptoms associated with varicose veins of lower extremities wherein the composition contains eicosapentaenoic acid.

2. The composition according to claim 1 wherein the composition is a food, a dietary suppliment, a cosmetic, or a quasi drug.

3. A prophylactic and therapeutic agent for varicose veins of lower extremities which contains eicosapentaenoic acid as its effective component.

4. The prophylactic and therapeutic agent for varicose veins of lower extremities according to claim 3 wherein the effective component is ethyl ester of the eicosapentaenoic acid.

5. Use of eicosapentaenoic acid in the manufacture of a prophylactic and therapeutic agent for varicose veins of lower extremities.

6. A method for preventing and treating varicose veins of lower extremities by using eicosapentaenoic acid.
